# EUROPEAN PATENT APPLICATION

(11) **EP 4 749 273 A1**
(43) Date of publication of application: **27.05.2026**
(21) Application number: 24826246.1
(22) Date of filing: 19.06.2024
(51) Int. Cl.: G01N 27/02, G01N 33/483

(54) **CELL CHIP FOR MEASURING CELL CHARACTERISTICS AND MANUFACTURING METHOD THEREFOR**

(30) Priority: 19.06.2023 KR 20230078450
(71) Applicant: Cellames Inc., Seongnam-si, Gyeonggi-do 13646 (KR)
(72) Inventor: CHO, Sang Jun, Seogwipo-si, Jeju-do 63519 (KR); PARK, Jinsoo, Anyang-si, Gyeonggi-do 14046 (KR)
(74) Representative: Caspary, Karsten
(86) International application number: PCT/KR2024/008461
(87) International publication number: WO 2024/262930

(57) **Abstract**

A cell chip for measuring cell characteristics according to an exemplary embodiment of the present disclosure may include a base substrate; a first polyimide film layer provided on an upper portion of the base substrate; a conductive pattern including a transmission line and an electrode pad, which is provided on the first polyimide film layer; and a second polyimide film layer provided with a pad hole exposing the electrode pad to cover an upper portion of the conductive pattern so as to insulate the transmission line from being exposed to the outside, thereby providing a cell chip that can be produced using a PCB process with relatively low manufacturing cost while ensuring biosafety as well as improving the accuracy and sensitivity in measuring cell characteristics.

## Description

### TECHNICAL FIELD

One embodiment of the present disclosure relates to a cell chip for measuring cell characteristics.

### BACKGROUND ART

The inventor of the present disclosure has conducted research and development to use an ICE (Interdigitated Carbon Electrode) electrode with an improved measurement sensitivity as electric cell-substrate impedance sensing (ECIS), and technology related to measuring a cell growth impedance according to the electrode structure of an ICE sensor dedicated to ECIS is introduced in Patent Document 1.

Conventional general cell chips have used base substrates such as biocompatible material PET, PC, glass, and silicon wafers, and insulating materials such as SU8 and silicon oxide series, but, in order to incorporate these materials into the production of cell chips, research and development is underway through an attempt to approach a printed circuit board (PCB) process method, which is relatively easy to access, due to issues such as high semiconductor process costs, minimum mass production quantities, and quality assurance methods. However, the production of cell chips incorporating the PCB process has difficulties such as difficulty in applying biocompatible material processes, limitations in producing the minimum implementable electrode pad structure, durability issues in specific environments (humidity of 90% or more), and decreased accuracy in measuring cell characteristics due to the absence of criteria for determining usability. Cell chips produced using conventional PCB process technology were implemented by a method of forming an insulating layer with a conductive pattern such as copper (Cu), nickel (Ni) or the like and a PSR on a base substrate made of materials such as FR-4, which is classified as having unknown biocompatibility, but those cell chips had problems in causing errors in measurement results due to the effect of the material on living organisms, the effect of leakage current, restrictions on use in high-humidity environments, and the structure of the electrode pad and variations in the length of the transmission line.

(Patent Document 1) KR 10-2018-0024388 A

### DISCLOSURE OF INVENTION

### TECHNICAL PROBLEM

An aspect of the present disclosure is to provide a cell chip for measuring cell characteristics with improved accuracy of measurement while reducing manufacturing costs and securing biosafety.

Another aspect of the present disclosure is to provide a method for manufacturing a cell chip for measuring cell characteristics with improved accuracy of measurement while reducing manufacturing costs and securing biosafety.

Still another aspect of the present disclosure is to provide a wave-shaped electrode structure having a uniform measurement sensitivity and an improved electric field magnitude and a method for determining usable suitability.

### TECHNICAL SOLUTION

A cell chip for measuring cell characteristics according to an exemplary embodiment of the present disclosure may include a base substrate; a first polyimide film layer provided on an upper portion of the base substrate; a conductive pattern including a transmission line and an electrode pad, which is provided on the first polyimide film layer; and a second polyimide film layer provided with a pad hole exposing the electrode pad to cover an upper portion of the conductive pattern so as to insulate the transmission line from being exposed to the outside.

In this case, preferably, the electrode pad may have an interdigitated structure in which a first electrode having a plurality of branch lines; and a second electrode having a plurality of branch lines in a direction opposite to the first electrode are interconnected with each other, wherein the branch lines of the first electrode and the second electrode have a wave shape.

Furthermore, the conductive pattern may further include a contact pad electrically connected to the transmission line, and preferably, the transmission line, the electrode pad, and the contact pad may be provided in plurality, one of the electrode pads may be connected to one of the contact pads by one of the transmission lines, and for each of the transmission lines, a width of the transmission may be determined such that a value obtained by dividing a length of the transmission line by the width of the transmission line is the same.

Furthermore, preferably, the base substrate may be made of an FR-4 material.

A method for manufacturing a cell chip for measuring cell characteristics according to an exemplary embodiment of the present disclosure may include forming a first polyimide film layer on an upper portion of a base substrate; forming a conductive pattern including a transmission line and an electrode pad on the first polyimide film layer; and bonding a second polyimide film layer having a pad hole exposing the electrode pad to an upper portion of the conductive pattern.

In this case, preferably, the second polyimide film layer may be bonded by a hot pressing method.

### ADVANTAGEOUS EFFECTS

According to one embodiment of the present disclosure, there may be provided a cell chip that can be produced using a PCB process with relatively low manufacturing cost while ensuring biosafety. Furthermore, the accuracy and sensitivity in measuring cell characteristics may be improved. Furthermore, suitability for use may be checked through the measurement of an electrolyte solution.

### BRIEF DESCRIPTION OF THE DRAWINGS

FIG. 1 is a diagram schematically illustrating a cell chip for measuring cell characteristics according to one embodiment of the present disclosure;
FIG. 2 is a diagram for explaining a conductive pattern of a cell chip for measuring cell characteristics according to one embodiment of the present disclosure;
FIG. 3 is a diagram for explaining an electric field of a cell chip for measuring cell characteristics according to one embodiment of the present disclosure;
FIG. 4 is a flowchart schematically illustrating a method for manufacturing a cell chip for measuring cell characteristics according to one embodiment of the present disclosure; and
FIG. 5 is a diagram for explaining a process for manufacturing a cell chip for measuring cell characteristics according to one embodiment of the present disclosure.

### BEST MODE FOR CARRYING OUT THE INVENTION

Advantages and features of the present disclosure, and methods of accomplishing the same will be clearly understood with reference to the following embodiments described below in detail in conjunction with the accompanying drawings. However, the present disclosure is not limited to the embodiments which will be disclosed below but may also be implemented in various different forms. The embodiments may be provided to complete the present disclosure and to allow those skilled in the art to fully understand the category of the disclosure Throughout the specification, the same reference numerals may represent the same elements.

It should be noted that the terms used herein are merely used to describe the embodiments, but not to limit the present disclosure. In this specification, unless clearly used otherwise, expressions in a singular form include a plural form. The term "comprise" and/or "comprising" used in the specification intend to express an element, a step, an operation and/or a device does not exclude the existence or addition of one or more other elements, steps, operations and/or devices.

All terms used herein, unless otherwise defined, have the same meaning as commonly understood by those skilled in the art to which the present disclosure pertains. The terms defined in commonly used dictionaries should be interpreted to have meanings identical to those used in the context of the related art and may not be interpreted to have ideal or excessively formal meanings unless clearly defined herein.

Hereinafter, the configuration and operational effects of the present disclosure will be described in more detail with reference to the accompanying drawings.

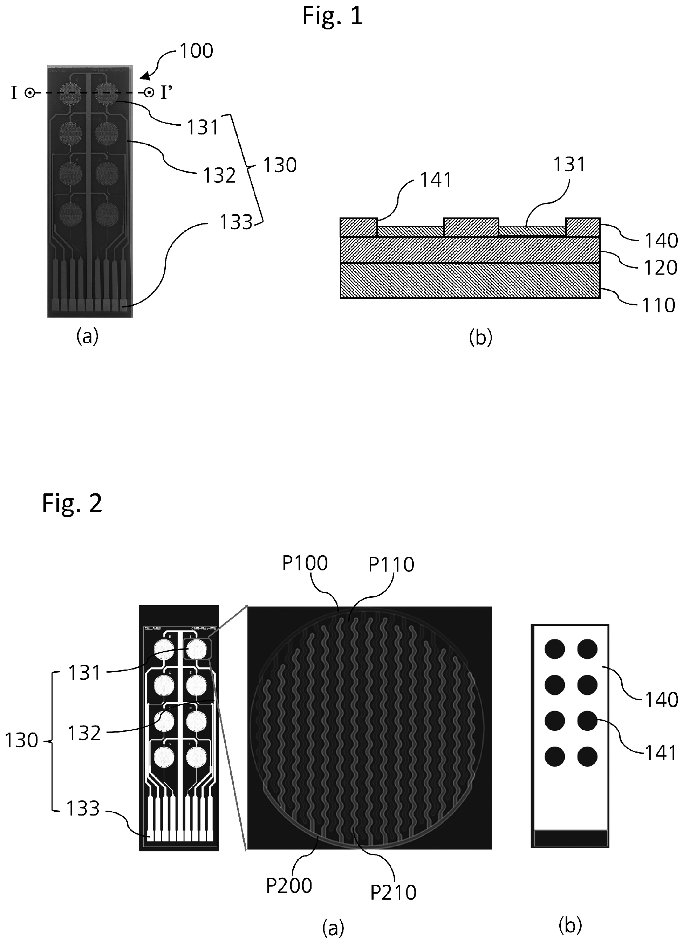

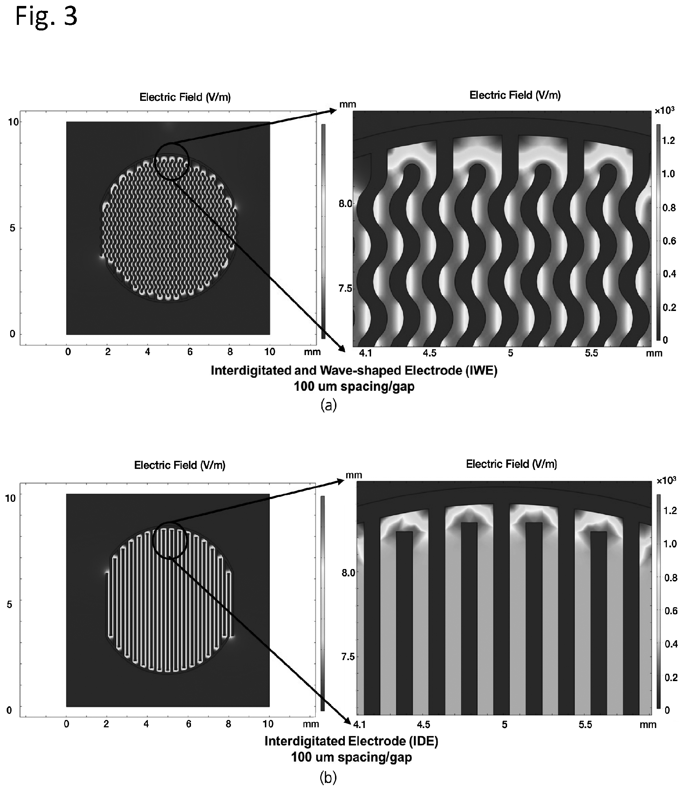

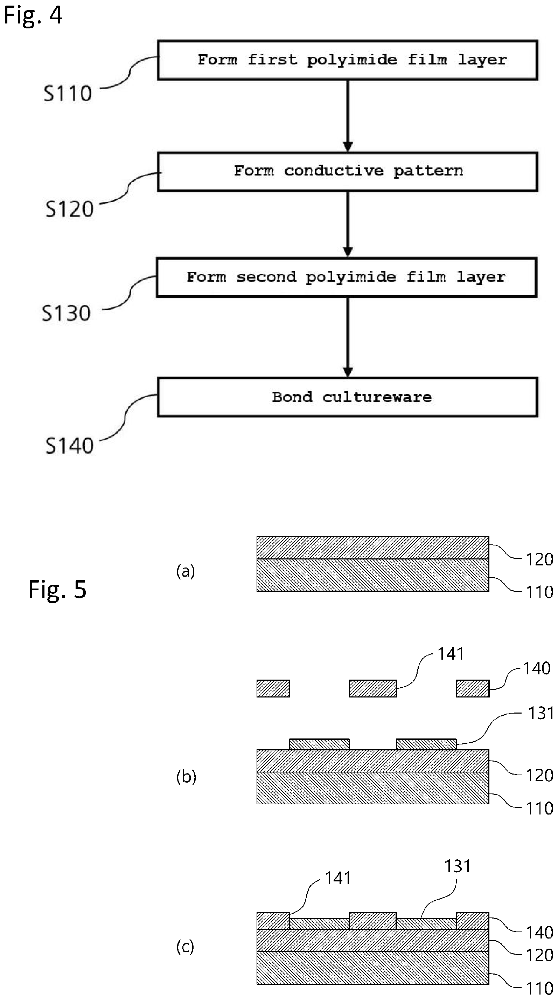

FIG. 1 is a diagram schematically illustrating a cell chip 100 for measuring cell characteristics according to one embodiment of the present disclosure, FIG. 2 is a diagram for explaining a conductive pattern 130 of the cell chip 100 for measuring cell characteristics according to one embodiment of the present disclosure, FIG. 3 is a diagram for explaining an electric field of the cell chip 100 for measuring cell characteristics according to one embodiment of the present disclosure, FIG. 4 is a flowchart schematically illustrating a method for manufacturing the cell chip 100 for measuring cell characteristics according to one embodiment of the present disclosure, and FIG. 5 is a diagram for explaining a process for manufacturing the cell chip 100 for measuring cell characteristics according to one embodiment of the present disclosure.

The cell chip 100 for measuring cell characteristics according to one embodiment of the present disclosure includes a base substrate 110, a first polyimide film layer 120, a conductive pattern 130, and a second polyimide film layer 140.

In one embodiment, the base substrate 110 may be made of an FR-4 material, which is widely utilized in producing a PCB. Meanwhile, according to one embodiment of the present disclosure, the first polyimide film layer 120, the second polyimide film layer 140, and the like may be bonded by a hot pressing method, and thus, the base substrate 110 may be preferably implemented with a material having a good temperature characteristic (relatively high temperature that can respond to the characteristic change initiation temperature of 180° C) and a strong bonding strength such that there is no peeling due to external factors after bonding.

In one embodiment, the first polyimide film layer 120, the second polyimide film layer 140, and the like may be bonded by a method of utilizing a silicone tape or a UV curing agent.

In one embodiment, the first polyimide film layer 120 is provided on an upper portion of the base substrate 110.

In one embodiment, the conductive pattern 130 is implemented on the first polyimide film layer 120. In this case, the conductive pattern 130 may include a transmission line 132 and an electrode pad 131. Moreover, the conductive pattern 130 may also include a contact pad 133.

In one embodiment, the contact pad 133 may perform a function that allows another device that receives a signal or current to be electrically connected to the cell chip. Additionally, the transmission line 132 may perform a function of electrically connecting between the electrode pad 131 and the contact pad 133.

In one embodiment, the electrode pad 131 may include a first electrode P100 and a second electrode P200 and may perform a function of coming into contact with a cell to detect an electrical signal of the cell. In this case, the first electrode and the second electrode may each have a plurality of branch lines, and each of the first electrode and the second electrode may have a comb structure. Furthermore, branch lines P110 of the first electrode and branch lines P210 of the second electrode may be arranged to interconnect with one another, but a predetermined gap may be provided between the branch lines of the first electrode and the branch lines of the second electrode. An electrode having this structure may be called an interdigitated electrode.

In one embodiment, the branch lines of the first electrode and the second electrode may be implemented in a wave shape. Referring to FIG. 4, compared to a case where the branch lines form a square shape, an edge-effect phenomenon in which the current is concentrated at the corners may be alleviated as the branch lines are implemented in a wave shape, and as a result, the uniformity of the electric field may be improved, the measurement sensitivity of cell characteristics may also be improved, and the problem of deviation in measurement results may be alleviated.

In one embodiment, a plurality of contact pads 133 may be provided on one side of the cell chip, and a plurality of electrode pads 131 may be provided on the other side of the cell chip. In this case, since the cells come into contact with a surface of the electrode pad 131, each of the plurality of electrode pads 131 must be provided at positions spaced apart from one another and occupies a predetermined area. Accordingly, a distance between each electrode pad 131 and each contact pad 133 implemented in one cell chip becomes different.

In one embodiment, one electrode pad 131 and one contact pad 133 may be connected by one transmission line 132. Additionally, lengths of respective transmission lines 132 vary depending on an arrangement structure or shape of the transmission line 132 connecting between the electrode pad 131 and the contact pad 133. Furthermore, the lengths of the respective transmission lines 132 may be determined by which path to connect the electrode pad 131 and the contact pad 133 within the cell chip. In this case, even if resistances of the respective transmission lines 132 are different in length, a width of the transmission line 132 may be determined so as to allow a deviation of the resistances of the respective transmission lines 132 to be within a predetermined allowable range.

For example, electrode pad #1 131 and contact pad #1 133 may be connected by transmission line #1 132, electrode pad #2 131 and contact pad #2 133 may be connected by transmission line #2 132, and the lengths of the transmission line #1 132 and the transmission line #2 132 may be different. In addition, even if the lengths of the transmission line #1 132 and the transmission line #2 132 are different, the widths of the transmission line #1 132 and the transmission line #2 132 may be determined so as to minimize a difference in resistance between the transmission line #1 132 and the transmission line #2 132. That is, since a resistance of the transmission line 132 is proportional to a length of the transmission line 132 and inversely proportional to a width of the transmission line 132, a resistance difference between the transmission line #1 132 and the transmission line #2 132 may be minimized by determining the widths of the transmission line #1 132 and the transmission line #2 132 so as to allow a value obtained by dividing the length of each transmission line 132 by the width of the transmission line 132 to be the same.

Tables 1 and 2 are diagrams for explaining a method for evaluating suitability of using a cell chip for measuring cell characteristics according to one embodiment of the present disclosure.

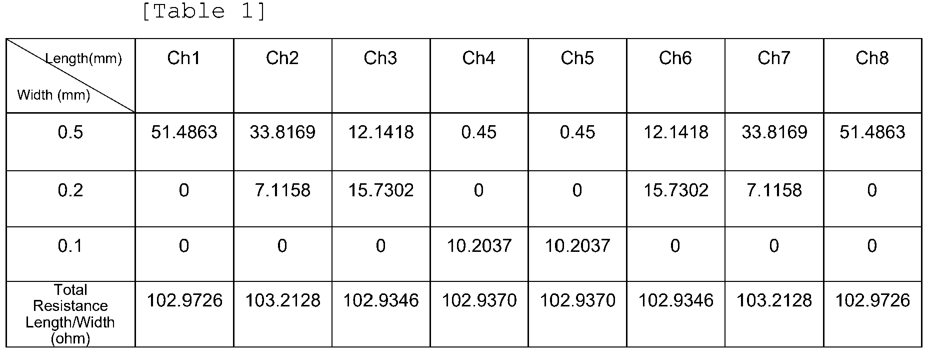

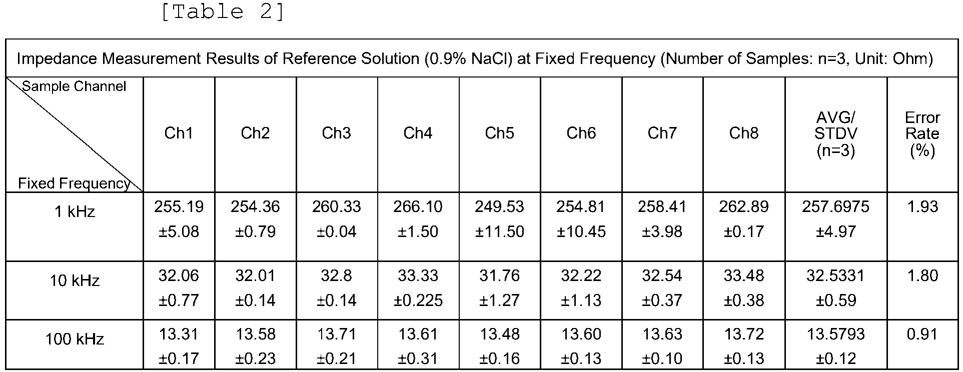

In one embodiment, for each transmission line, a width of transmission line may be determined so as to allow a value obtained by dividing a length of the transmission line by the width of the transmission line to be the same. In another embodiment, a value obtained by dividing a length of the transmission line by a width thereof may be in a range of 50 to 300 ohm.

In one embodiment, a reference electrolyte solution (0.9% NaCl) may be provided to the electrode pads, and impedance measurement values according to a fixed frequency may be within a range of 200 to 300 Ohm at 1 kHz, 30 to 50 ohm at 10 kHz, and 1 to 20 ohm at 100 kHz with an error rate of within 10% for each of the plurality of electrode pads so as to verify suitability for use. In this case, a NaCl solution may be utilized as the electrolyte solution, and it is preferable to prepare and utilize a concentration of 0.9% in consideration of the composition of cell culture medium and sodium concentration in the body.

In one embodiment, the second polyimide film layer 140 is provided on an upper portion of the conductive pattern 130. In this case, the second polyimide film layer 140 is provided with a pad hole 141, and the electrode pads 131 described above may be exposed through this pad hole 141 while insulating the transmission lines 132. In one embodiment, the pad hole may have a shape such as a circle, an oval, a polygon, or the like, and may be formed to correspond to the shape of the electrode pad 131. In one embodiment, when the pad hole is formed in a circular shape, its diameter may be in a range of 1 mm to 10 mm.

Accordingly, an electric signal of a cell coming into contact with the first electrode pad 131 may be output only to the first contact pad 133 via the first transmission line 132, and may be prevented from affecting other electrode pads 131, other transmission lines 132, and other contact pads 133 such as the second electrode pad 131, the second transmission line 132, and the second contact pad 133.

According to one embodiment of the present disclosure, the first polyimide film layer 120 is provided on an upper surface of the base substrate 110, the conductive pattern 130 is provided on an upper surface of the first polyimide film layer 120, and the second polyimide film layer 140 covers part of the conductive pattern 130, wherein a portion of the electrode pad 131 is opened through the pad hole 141 of the second polyimide film layer 140.

Here, the material such as FR-4 that implements the base substrate 110 has not only not been fully verified for its effect on living organisms, but the base substrate 110 itself is vulnerable to moisture and has the characteristic of absorbing moisture, and therefore, when a culture material including moisture comes into contact with the base substrate 110 in a long-term weekly high-humidity environment during cell characteristic analysis, a minute leakage current may occur between the transmission lines 132, which may cause an error in the process of measuring the electrical characteristics of the cell. Additionally, a photo solder resist (PSR), which is widely used in a general PCB or the like, is also a material whose effects on living organisms have not been fully verified. Therefore, when producing cell chips using existing general PCB-related technologies, there is a concern that the measurement results may differ due to the effects of materials such as FR-4 and PSR on living organisms. However, in the cell chip 100 for measuring cell characteristics according to one embodiment of the present disclosure, the conductive pattern 130 may be implemented on the first polyimide film layer 120 so as to significantly reducing a change in the characteristics of a living organism due to the material of the cell chip or a measurement error due to leakage current.

Referring to FIG. 4, a method for manufacturing the cell chip 100 for measuring cell characteristics according to one embodiment of the present disclosure may include forming the first polyimide film layer 120 (S110), forming the conductive pattern 130 (S120), and forming the second polyimide film layer 140 (S130), and if necessary, may further include bonding cultureware (S140).

In one embodiment, the first polyimide film layer 120 may be formed on an upper portion of the base substrate 110. In one embodiment, a film of polyimide material may be bonded to the base substrate 110 by a hot pressing method to form the first polyimide film layer 120.

In one embodiment, the conductive pattern 130 may be formed on the first polyimide film layer 120. In one embodiment, the conductive pattern 130 may be performed by an electroless/electrolytic plating method.

In one embodiment, the conductive pattern may be made of a Cu/Ni/Au material.

In one embodiment, a thickness of the conductive pattern may be determined in a range of 100 to 1000 nm.

In one embodiment, the second polyimide film layer 140 may be formed to cover the conductive pattern 130. In one embodiment, the pad hole 141 may be formed on a film made of a polyimide material, and then bonded to an upper portion of the conductive pattern 130 by a hot pressing method to form the second polyimide film layer 140.

In one embodiment, the first polyimide film layer 120, the second polyimide film layer 140, and the like may be bonded by a method of utilizing a silicone tape or a UV curing agent.

In one embodiment, cultureware that defines an area corresponding to each of the plurality of electrode pads 131 may be bonded to an upper portion of the cell chip 100 for measuring cell characteristics.

In the above, although representative embodiments of the present disclosure have been described in detail, those skilled in the art will understand that various modifications can be made to the foregoing embodiments without departing from the scope of the present disclosure. Therefore, the scope of the present disclosure should not be limited to the described embodiments, but should be defined not only by the claims described below but also by equivalents to the claims.

### DESCRIPTION OF SYMBOLS

- 100:: Cell chip for measuring cell characteristics
- 110:: Base substrate
- 120:: First polyimide film layer
- 130:: Conductive pattern
- 131:: Electrode pad
- 132:: Transmission line
- 133:: Contact pad
- 140:: Second polyimide film layer
- 141:: Pad hole

### INDUSTRIAL APPLICABILITY

A cell chip according to one embodiment of the present disclosure may be used in the bio-industry field for analyzing characteristics of a cell. In particular, cell chips can be used for non-destructive real-time analysis, such as cell impedance measurement and local field potential measurement for 2D/3D cell analysis and toxicity evaluation.

## Claims

1. A cell chip for measuring cell characteristics, the cell chip comprising:
a base substrate;
a first polyimide film layer provided on an upper portion of the base substrate;
a conductive pattern comprising a transmission line and an electrode pad, which is provided on the first polyimide film layer; and
a second polyimide film layer provided with a pad hole exposing the electrode pad to cover an upper portion of the conductive pattern so as to insulate the transmission line from being exposed to the outside.

2. The cell chip of claim 1, wherein the electrode pad has an interdigitated structure in which a first electrode having a plurality of branch lines; and a second electrode having a plurality of branch lines in a direction opposite to the first electrode are interconnected with each other, and
wherein the branch lines of the first electrode and the second electrode have a wave shape.

3. The cell chip of claim 1, wherein the conductive pattern further comprises a contact pad electrically connected to the transmission line,
wherein the transmission line, the electrode pad, and the contact pad are provided in plurality,
wherein one of the electrode pads is connected to one of the contact pads by one of the transmission lines, and
wherein for each of the transmission lines, a width of the transmission is determined such that a value obtained by dividing a length of the transmission line by the width of the transmission line is the same.

4. The cell chip of claim 1, wherein the base substrate is made of an FR-4 material.

5. A method for manufacturing a cell chip for measuring cell characteristics, the method comprising:
forming a first polyimide film layer on an upper portion of a base substrate;
forming a conductive pattern including a transmission line and an electrode pad on the first polyimide film layer; and
bonding a second polyimide film layer having a pad hole exposing the electrode pad to an upper portion of the conductive pattern.

6. The method of claim 5, wherein the second polyimide film layer is bonded by a hot pressing method.
